# EUROPEAN PATENT APPLICATION

(11) **EP 3 967 280 A1**
(43) Date of publication of application: **16.03.2022**
(21) Application number: 21174155.8
(22) Date of filing: 17.05.2021
(51) Int. Cl.: A61F 2/91, A61F 2/915, A61F 2/82, A61F 2/95, A61F 2/966

(54) **SYSTEMS AND METHODS FOR TREATMENT OF DEFECTS IN THE VASCULATURE**

(30) Priority: 15.05.2020 US 202063025693 P
(71) Applicant: DePuy Synthes Products, Inc., Raynham, MA 02767 (US)
(72) Inventor: KOTTENMEIER, Emilie, Raynham, 02767 (US); LEE, Stephanie Hsiao Yu, Raynham, 02767 (US); KHANNA, Rahul, Raynham, 02767 (US)
(74) Representative: Carpmaels & Ransford LLP

(57) **Abstract**

Methods and systems including an expandable stent and achieving, by the expandable stent, a reduction in odds of 12-month inpatient readmission and/or a reduction in total cost of care for a plurality of patients.

## Description

### CROSS-REFERENCE TO RELATED APPLICATIONS

This application claims the benefit of U.S. Provisional Application Serial No. 63/025,693, filed on 15 May 2020, which is incorporated herein by reference in its entirety as if fully set forth below.

### FIELD

This disclosure relates generally to devices for interventional therapeutic treatment or vascular surgery for treatment of defects in the vasculature.

### BACKGROUND

Intracranial saccular aneurysms are common acquired lesions. Several stent options are currently available to treat unruptured intracranial aneurysms (UIA). Several risk factors affecting both aneurysm formation/growth and risk of rupture have been identified: aneurysm shape and location, gender, age, family history, genetic conditions, cigarette smoking, hypertension, alcohol consumption and drug use. Occurrence of intracranial saccular aneurysms is increased in some inherited disorders compared with the general population. Autosomal dominant polycystic kidney (ADPKD) disease is the most common inherited disorder associated with intracranial saccular aneurysm, though other common disorders are known to be linked to intracranial saccular aneurysm.

The inherent threat of a UIA is a rupture and subsequent subarachnoid hemorrhage (SAH). Intracranial aneurysms have to be considered as an important disease due to the severe morbidity and mortality associated with SAH that result from rupture of cerebral aneurysms. The most important objective of the treatment is to prevent rupture or rebleeding by isolating an aneurysm from the normal blood circulation without narrowing the parent vessel. Long term stability of the occlusion of the aneurysmal sac after initial treatment is a desirable outcome as recanalization, or neck re-growth can make the aneurysm more susceptible to rupture and can result in a negative neurological impact as the blood flow is misdirected into the aneurysm and not into the appropriate circulation. Simply put, if left untreated, aneurysms may continue to grow or rupture. Risk levels and rates of these types of events vary based on the location of the aneurysm as previously discussed.

Existing stents have been investigated in single-center, single-arm studies. Yet, fewer studies have direct comparisons of stent utility or effectiveness. Few studies have compared outcomes among patients with UIA undergoing stent-assisted coiling and these studies have reported mixed results. The solution of this disclosure resolves these and other issues of the art.

### SUMMARY

The subject of this disclosure includes methods or uses of an expandable stent to treat aneurysms in the neurovascular arteries and veins as well as other vascular beds.

In some examples, a method or use is disclosed that includes delivering an expandable stent to within a blood vessel adjacent an aneurysm of a plurality of human patients; delivering one or more coils to the aneurysm; and achieving, by the expandable stent, approximately 20% 12-month all-cause inpatient readmission for the plurality of human patients.

In some examples, a method or use is disclosed that includes delivering an expandable stent to within a blood vessel adjacent an aneurysm of a plurality of human patients; delivering one or more coils to the aneurysm; and; and achieving, by the expandable stent, approximately 15% 12-month cardiovascular related inpatient readmission for the plurality of human patients.

In some examples, a method or use is disclosed that includes delivering an expandable stent to within a blood vessel adjacent an aneurysm of a plurality of human patients; delivering one or more coils to the aneurysm; and; and achieving, by the expandable stent, approximately 10% 12-month unruptured intracranial aneurysm related inpatient readmission for the plurality of human patients.

In some examples, a method or use is disclosed that includes delivering an expandable stent to within a blood vessel adjacent an aneurysm in a plurality of human patients; delivering one or more coils to the aneurysm; and; and achieving, by the expandable stent, approximately $105,000 USD total cost of care for all-cause related readmission per human patient of the plurality of human patients.

In some examples, a method or use is disclosed that includes delivering an expandable stent to within a blood vessel adjacent an aneurysm of a plurality of human patients; delivering one or more coils to the aneurysm; and; and achieving, by the expandable stent, approximately $38,500 USD total cost of care for cardiovascular related inpatient readmission per human patient of the ROk plurality of human patients.

In some examples, a method or use is disclosed that includes delivering an expandable stent to within a blood vessel adjacent an aneurysm of a plurality of human patients; delivering one or more coils to the aneurysm; and; and achieving, by the expandable stent, approximately $37,600 USD total cost of care for unruptured intracranial aneurysm related inpatient readmission for the plurality of human patients.

In some examples, the plurality of human patients includes at least approximately 486 human patients from a plurality of different hospital sites.

In some examples, a method or use is disclosed that includes reducing odds of 12-month inpatient readmission by delivering an expandable stent to within a blood vessel adjacent an aneurysm of a plurality of human patients and delivering one or more coils to the aneurysm.

In some examples, the plurality of human patients divided into a first group treated by the expandable stent and a second group treated by a second clinically approved stent, the odds being 12-month all-cause readmission and being at least approximately 28% lower for the first group versus the second group.

In some examples, the plurality of human patients is divided into a first group treated by the expandable stent and a second group treated by a second clinically approved stent, the odds being 12-month cardiovascular related readmission and being at least approximately 30% lower for the first group versus the second group.

In some examples, the plurality of human patients is divided into a first group treated by the expandable stent and a second group treated by a second clinically approved stent, the odds being 12-month unruptured intracranial aneurysm related readmission and being at least approximately 38% lower for the first group versus the second group.

In some examples, the odds are determined by a generalized estimating equations (GEE) model.

In some examples, a method or use is disclosed that includes reducing a total cost of care for a plurality of human patients by delivering an expandable stent to within a blood vessel adjacent an aneurysm of a plurality of human patients and delivering one or more coils to the aneurysm.

In some examples, the plurality of human patients is divided into a first group treated by the expandable stent and a second group treated by a second clinically approved stent, the total cost of care for all-cause related readmission per human patient of the plurality of human patients being approximately $105,000 USD for the first group and approximately $127,000 USD for the second group.

In some examples, the plurality of human patients is divided into a first group treated by the expandable stent and a second group treated by a second clinically approved stent, the total cost of care for cardiovascular related inpatient readmission per human patient of the plurality of human patients being approximately $38,500 USD for the first group and approximately $42,800 USD for the second group.

In some examples, the plurality of human patients is divided into a first group treated by the expandable stent and a second group treated by a second clinically approved stent, the total cost of care for unruptured intracranial aneurysm related inpatient readmission per human patient of the plurality of human patients being approximately $37,600 USD for the first group and approximately $41,400 USD for the second group.

In some examples, the total cost of care is adjusted for medical inflation and reported in 2018 US dollars ($).

In some examples, inclusion criteria for the plurality of human patients are defined as patients who underwent an inpatient endovascular procedure for unruptured intracranial aneurysm between 2011-2018 were identified from the Premier Healthcare Database.

In some examples, inclusion criteria for the plurality of human patients are defined as patients≥ 18 years old at the time of index hospital admission.

In some examples, exclusion criteria for the plurality of human patients are defined as patients who had their index endovascular procedure at hospitals who did not continuously provide data to PHD in the 12-month pre-index period.

In some examples, the expandable stent includes at least one anchor member interlocked within at least one gap along a core member of a corresponding delivery system, the at least one gap formed by spaces between a plurality of cylindrical members disposed on the core member.

In some examples, the method or use includes mounting the expandable stent on at least one cylindrical member along the core member of the delivery system, the delivery system comprising a deployment catheter disposed about the stent maintaining the stent in a constrained configuration.

In some examples, the method or use includes moving the deployment catheter proximally allowing said stent to begin expanding within the vessel.

In some examples, the method or use includes further moving the deployment catheter proximally allowing the stent to further deploy causing the vessel to increase luminal dilation.

In some examples, the method or use includes removing said delivery system from said blood vessel.

To the accomplishment of the foregoing and related ends, certain illustrative aspects are described herein in connection with the following description and the appended drawings. These aspects are indicative, however, of but a few of the various ways in which the principles of the claimed subject matter may be employed and the claimed subject matter is intended to include all such aspects and their equivalents. Other advantages and novel features may become apparent from the following detailed description when considered in conjunction with the drawings.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
FIG. 1 is a perspective view of an expandable stent evaluated in a study of this disclosure.
FIG. 2 is a flow diagram illustrating flow of enrollment in the study of this disclosure.
FIG. 3 is a table summarizing mean differences before and after matching in the primary outcome cohort.
FIG. 4 is a table summarizing mean differences before and after matching in the twelve -month outcome cohort.
FIG. 5 is a table summarizing rates of twelve-month inpatient readmission between groups of the study.
FIG. 6 is a table summarizing twelve-month costs of care for index admission and inpatient readmission between groups of the study.
FIG. 7 is a table summarizing rates of complications between groups of the study.
FIG. 8 depicts a graphical overview of one method of treating an aneurysm according to this disclosure.
FIG. 9 depicts a graphical overview of a method of treating an aneurysm according to this disclosure.
FIG. 10 depicts a graphical overview of a method of treating an aneurysm according to this disclosure.
FIG. 11 depicts a graphical overview of a method of treating an aneurysm according to this disclosure.
FIG. 12 depicts a graphical overview of a method of treating an aneurysm according to this disclosure.
FIG. 13 depicts a graphical overview of a method of treating an aneurysm according to this disclosure.
FIG. 14 depicts a graphical overview of a method of treating an aneurysm according to this disclosure.
FIG. 15 depicts a graphical overview of a method of treating an aneurysm according to this disclosure.

### DETAILED DESCRIPTION

Although example embodiments of the disclosed technology are explained in detail herein, it is to be understood that other embodiments are contemplated. Accordingly, it is not intended that the disclosed technology be limited in its scope to the details of construction and arrangement of components set forth in the following description or illustrated in the drawings. The disclosed technology is capable of other embodiments and of being practiced or carried out in various ways.

It must also be noted that, as used in the specification and the appended claims, the singular forms "a," "an" and "the" include plural referents unless the context clearly dictates otherwise. By "comprising" or "containing" or "including" it is meant that at least the named compound, element, particle, or method step is present in the composition or article or method, but does not exclude the presence of other compounds, materials, particles, method steps, even if the other such compounds, material, particles, method steps have the same function as what is named.

In describing example embodiments, terminology will be resorted to for the sake of clarity. It is intended that each term contemplates its broadest meaning as understood by those skilled in the art and includes all technical equivalents that operate in a similar manner to accomplish a similar purpose. It is also to be understood that the mention of one or more steps of a method does not preclude the presence of additional method steps or intervening method steps between those steps expressly identified. Steps of a method may be performed in a different order than those described herein without departing from the scope of the disclosed technology. Similarly, it is also to be understood that the mention of one or more components in a device or system does not preclude the presence of additional components or intervening components between those components expressly identified.

As discussed herein, vasculature of a "subject" or "patient" may be vasculature of a human or any animal. It should be appreciated that an animal may be a variety of any applicable type, including, but not limited to, mammal, veterinarian animal, livestock animal or pet type animal, etc. As an example, the animal may be a laboratory animal specifically selected to have certain characteristics similar to a human (e.g., rat, dog, pig, monkey, or the like). It should be appreciated that the subject may be any applicable human patient.

As discussed herein, "operator" may include a doctor, surgeon, or any other individual, medical interventionalist, or delivery instrumentation associated with delivery of a flow diverter device, and related instrumentation, to an aneurysm in the vasculature of a subject.

The terms "distal" or "proximal" are used in the following description with respect to a position or direction relative to the treating physician or medical interventionalist. "Distal" or "distally" are a position distant from or in a direction away from the physician or interventionalist. "Proximal" or "proximally" or "proximate" are a position near or in a direction toward the physician or medical interventionist.

As discussed herein, the term "rate" as used throughout this disclosure is intended to refer to the rate for a particular population of patients according to a particular investigation rather than information or levels related to a single patient. However, herein the term "rate" and "level" can be used interchangeably. In any study, single patient levels are used to determine "rates." Any one patient's level may be the notable point in a reported rate.

As used herein, the terms "about" or "approximately" for any numerical values or ranges indicate a suitable tolerance. More specifically, "about" or "approximately" can refer to the range of values ±20% of the recited value, e.g. "about 90%" can refer to the range of values from 71% to 99%.

As discussed herein, the term "safety", as it relates to a self-expanding braided stent, delivery system, or method of treatment refers to a relatively low severity of adverse events, including adverse bleeding events, infusions, or hypersensitivity reactions. Adverse bleeding events can be the primary safety endpoint and include, for example, major bleeding, minor bleeding, and the individual components of the composite endpoint of any bleeding event.

As discussed herein, unless otherwise noted, the term "clinically effective" (used independently or to modify the term "effective") can mean that it has been proven by a clinical trial wherein the clinical trial has met the approval standards of U.S. Food and Drug Administration (FDA), EMEA or a corresponding national regulatory agency. For example, a clinical study may be an adequately sized, randomized, double-blinded controlled study used to clinically prove the effects of the reperfusion device and related systems of the disclosure. Most preferably to clinically prove the effects of the device with respect to an aneurysm, for example, to achieve a clinically effective outcome for the patient with the aneurysm (e.g., mRS less than or equal to 2) and/or achieve reperfusion of the vessel(s) afflicted by the aneurysm subject to treatment.

As discussed herein, the term "computed tomography" or "CT" means one or more scans that make use of computer-processed combinations of two or more X-ray measurements taken from different angles to produce cross-sectional (tomographic) images (virtual "slices") of specific areas of a scanned object, allowing the user to see inside the object without cutting. Such CT scans of this disclosure can refer to X-ray CT as well as many other types of CT, such as positron emission tomography (PET) and single-photon emission computed tomography (SPECT).

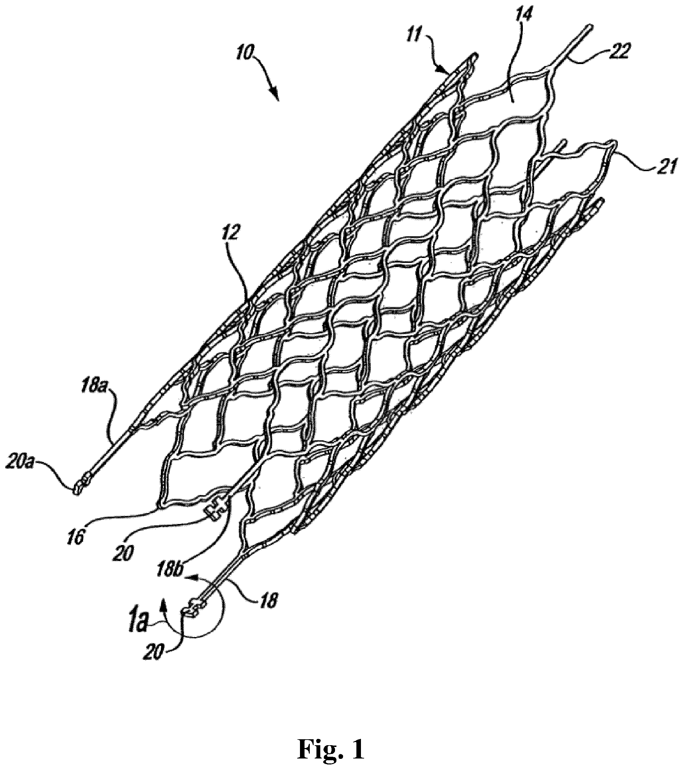

The present disclosure is related to systems, methods, and devices for treating hemorrhagic events and restoring perfusion in blood vessels. FIG. 1 illustrates an example self-expanding stent 10, which was evaluated in the study of this disclosure and can be understood as including features more clearly described in Appendix 1 of provisional application number 63/025,693 filed on 15 May 2020, which includes U.S. Pat. No. 6,612,012, U.S. Pat. No. 6,955,685, U.S. Pat. No. 6,960,227, U.S. Pat. No. 6,833,003, U.S. Pat. No. 7,001,422, U.S. Pat. No. 7,195,648, U.S. Pat. No. 7,201,769, U.S. Pat. No. 7,309,351, U.S. Pat. No. 7,311,726, U.S. Pat. No. 7,037,331, U.S. Pat. No. 6,673,106, U.S. Pat. No. 6,818,013, U.S. Pat. No. 6,960,228, U.S. Pat. No. 8,182,523, U.S. Pat. No. 8,734,500, and U.S. Pat. No. 9,693,885, each of which are incorporated by reference in their entirety as if set forth verbatim herein. Stent 10 can be laser cut to form a thin-walled, skeletal tubular member 11 comprised of nickel-titanium alloy. Once cut, the wall of the tubular member 11 can include several openings, or cells 14. When the skeletal tubular member 11 is placed over an aneurysm, a physician is able to deliver embolic coils or other such devices through the cells 14 and into the aneurysm. The tubular member 11 also functions to cover the mouth of the aneurysm thus obstructing, or partially obstructing, the flow of blood into the aneurysm. Also, the tubular member 11 prevents medical devices such as embolic coils from escaping the aneurysm.

The preferred length of the skeletal tubular member 11 may range from 0.0795 inches to 3.15 inches. The diameter of the tubular member 11 varies depending on its deployment configuration. In a non-deployed or expanded state, the diameter of the tubular member 11 may extend up to about 0.4 inches. When the skeletal tubular member 11 is compressed to fit within the lumen of a deployment catheter, the diameter may be reduced to about 0.014 inches.

Attached to the proximal end 16 of the skeletal tubular member 11 are three proximal legs 18, 18a, and 18b that extend longitudinally from the tubular member 11. The proximal legs 18, 18a, and 18b are preferably biased outwardly from the longitudinal axis of the tubular member 11. This outwardly biased configuration aids in delivery. T-shaped or I-shaped attachment flanges 20, 20a, and 20b can be attached to the tips of each proximal leg 18, 18a, and 18b. Stent 10 can be used with one or more coils for insertion into the aneurysm and delivered by a delivery system, similar to the delivery system shown and described in U.S. Pat. No. 7,001,422, which is included in Appendix 1 of provisional application number 63/025,693 and is incorporated by reference in its entirety as if set forth verbatim herein. However, other catheter delivery systems are contemplated for use with stent 10.

### Study Overview

This disclosure is more clearly understood with a corresponding study discussed more particularly below with respect to treatment of hemorrhagic events, attached hereto in Appendix 2 of provisional application number 63/025,693 filed on 15 May 2020, and incorporated by reference in its entirety as if set forth verbatim herein. It is understood that data is presented herein for purposes of illustration and should not be construed as limiting the scope of the disclosed technology in any way or excluding any alternative or additional embodiments.

A primary objective of the study was to compare the short- and long-term clinical effectiveness and economic outcomes among human patients undergoing endovascular treatment for UIA with stent 10, which can include the Enterprise stent (CERENOVUS, Irvine, CA), and can have a closed-cell design to partially deploy, recapture, and redeploy the stent for improved treatment of wide-necked aneurysms. These effectiveness and economic outcomes were compared with those of the Neuroform stent system (Stryker Neurovascular, Kalamazoo, MI), as shown and described in U.S. Pat. No. 7,037,330 and U.S. Pat. No. 7,695,507, and the Low-profile Visualized Intraluminal Support (LVIS; MicroVention, Aliso Viejo, CA) stent. The Neuroform stent is a laser-cut nitinol stent with an open-cell design. The LVIS stent is a braided self-expanding nitinol stent with a closed-cell implant. These stents also differ in terms of available sizes, pore density, metal coverage, average loading force, passage force, and deployment force, all of which could potentially lead to differences in patient outcomes.

Though previous studies have provided useful information, the generalizability of their findings was limited by a single-center nature together with a lack of adjustment for patient characteristics in outcome assessments. To resolve the limitation, this study utilized a nationally representative hospital billing dataset to perform a retrospective cohort analysis comparing stents in terms of real-world clinical and economic outcomes after accounting for patient demographics, comorbidity, procedural, and provider characteristics differential. In particular, the study included a retrospective cohort analysis of hospital-level data from the Premier Healthcare Database (PHD) for patients who underwent an elective inpatient endovascular coiling procedure with the braided stent of this disclosure with a primary diagnosis of unruptured intracranial aneurysm (UIA) between January 1, 2011 and December 31, 2018. *See* Premier Applied Sciences, Premier Healthcare Database White Paper: Data that informs and performs. 2018*.* Data from the PHD used in this study contained clinical coding, hospital cost, and patient billing data from more than 700 hospitals throughout the United States. Although the database excluded federally funded hospitals (e.g., Veterans Affairs), the hospitals included were nationally representative based on bed size, geographic region, location (urban/rural), and teaching hospital status. The database contained a date-stamped log of all billed items by cost-accounting departments including medications; laboratory, diagnostic, and therapeutic services; and primary and secondary diagnoses for each patient's hospitalization. Identifier-linked enrollment files provided demographic and payor information. Detailed service-level information for each hospital day was recorded, including details about medications and devices.

This data was collected for the date of index hospitalization. Comorbidities were assessed using Charlson Comorbidity Index (CCI) scores. *See* de Groot V, Beckerman H, Lankhorst GJ, et al. How to measure comorbidity: a critical review of available methods. J Clin Epidemiol 2003;56:221-29. doi: 10.1016/S0895-4356(02)00585-1. The CCI is understood as an aggregate measure of comorbidity that combines 19 select diagnoses associated with chronic disease (e.g., heart disease, cancer) weighted on a scale from +1 to +6. Higher scores were indicative of greater comorbidity burden. Data was also controlled for specific comorbidities including diabetes, hypertension, polycystic kidney disease, congestive heart failure, peripheral vascular disorder, chronic pulmonary disease, ischemic stroke/transient ischemic attack (TIA), hypothyroidism, obesity, and depression.

The data also included hospital characteristics, including teaching status (teaching/non-teaching), geographic location (Midwest, Northeast, South, West), size (< 300 beds, 300-399 beds, 400-499 beds, and ≥ 500 beds), urban-rural classification, and volume of endovascular coil procedures for UIA (using a median split) in the 12-month pre-index period. With this data, the study analyzed primary outcomes, such as short-term clinical effectiveness measures including discharge status (defined in terms of mortality: died/alive) and length of stay (LOS) for the index admission, and long-term clinical effectiveness measure including 12-month inpatient readmission (all-cause, cardiovascular [CV]-related, UIA-related, SAH-related). The 12-month total inpatient costs (sum of index admission cost and 12-month inpatient readmission cost) was also assessed. Readmissions is a key marker of long-term treatment effectiveness when considering secondary data sources such as the PHD, which do not include tailored study endpoint variables. Moreover, readmissions for cardiovascular and UIA reasons are a clinically important measure of success for patients and healthcare providers.

All costs were adjusted for medical inflation and are reported in 2018 US dollars (USD). A secondary outcome of the study was complication rate, such as ischemic (e.g., aphasia, hemiplegia, or cerebral artery occlusion), hemorrhagic (e.g., SAH or intracerebral hemorrhage), neurologic, and other surgical complications (e.g., ventriculostomy, ventriculoperitoneal shunt surgery, or tracheostomy). As healthcare dollars are increasingly scrutinized, it is important to understand cost differences associated with device selection from both a payer and provider perspective.

All study variables are summarized as the mean and standard deviation for continuous variables and the frequency and percentage for categorical variables. Bivariate statistical tests (Chi square tests for categorical variables and t-tests for continuous variable comparison) were conducted to examine and describe between-group differences in potential confounding factors such as patient demographics, clinical characteristics, procedural characteristics, and provider characteristics.

### Patient Selection

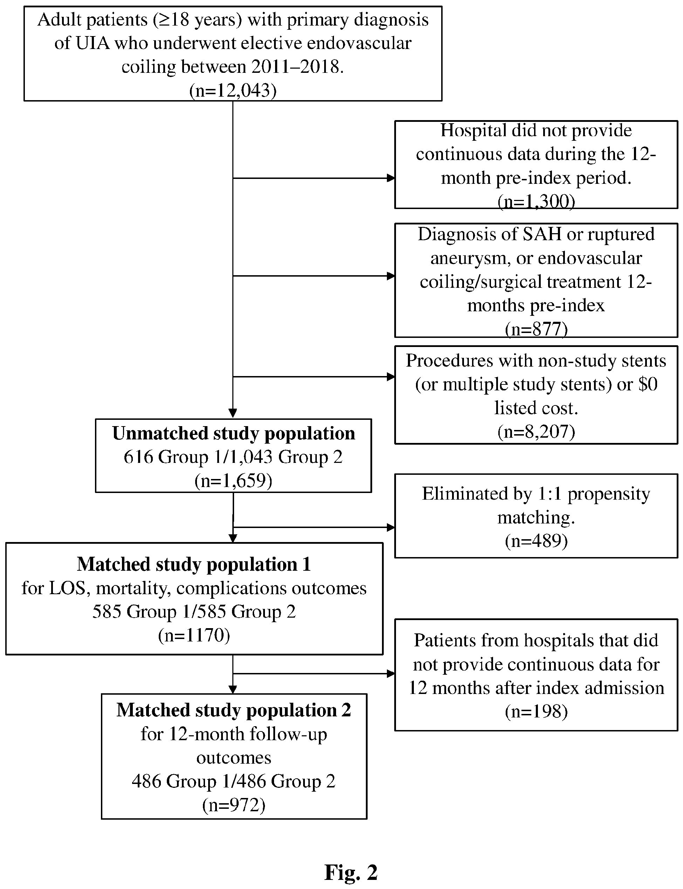

Patients of the study were those who underwent an inpatient endovascular procedure for UIA between 2011-2018 and identified from the Premier Healthcare Database. Patients were classified into treatment groups: (1) stent 10 or (2) Neuroform or LVIS stent (hereafter "Group 1" and "Group 2", respectively). The sequence of patient enrollment is shown in Fig. 2. Propensity score matching was used to match patients in Group 1 with those in Group 2 based on study covariates. Patients were matched 1:1 using the GREEDY match nearest neighbor technique without replacement, enforcing a caliper of 0.10. Post-match balance of covariates was examined using standardized mean differences (SMDs): a SMD <0.10 was used to indicate good balance. SMDs were used as opposed to Chi square coefficients as the former provide covariate balance information independent of sample size.

Two different propensity matching procedures were performed: (1) to compare outcomes associated with index admission (i.e., LOS, discharge status, and complications), propensity matching was performed on the entire pre-match sample identified based on study eligibility criteria; (2) for outcomes based on 12-month follow-up assessment (i.e., 12-month all-cause, CV-related, and UIA-related inpatient readmission, and total admission cost [sum of index admission and 12-month all-cause inpatient readmissions]), patients were only matched if they had index admission at hospitals that continuously provided data to the PHD for 12 months after the index hospital admission.

Bivariate analyses were conducted using student's t-test or Chi square/Fisher's exact test. Generalized estimating equation (GEE) models with an exchangeable correlation structure and appropriate link (logit link for discharge status, complications, and readmission comparison; log link for length of stay (LOS) and cost comparison) and distribution function (binomial distribution for discharge status, complications, and readmission; negative binomial distribution for LOS; gamma distribution for cost) were utilized to compare outcomes between the study groups. GEE analyses adjusted comparisons for hospital clustering and any covariate that emerged significant post-matching (i.e., SMD ≥0.10 or ≤-0.10). Outcomes investigated included length of stay (LOS), discharge status (mortality), 12-month inpatient readmission, and total cost of care (index admission plus 12-month all-cause readmission). Groups 1 and 2 were propensity-score matched and generalized estimating equations (GEE) were used for outcomes assessment, adjusting for hospital clustering.

Of these groups, there were approximately 585 matched patients per group. There were no significant between-group differences in LOS (adjusted ratio of the mean 0.90, 95% confidence interval [95%CI] 0.73-1.10) or discharge status (adjusted ratio of the mean 1.98, 95%CI 0.48-8.07) between Groups 1 and 2, respectively. Group 1 had significantly lower odds of 12-month all-cause (odds ratio [OR] 0.72, 95%CI 0.53-0.97), cardiovascular-related (OR 0.70, 95%CI 0.50-0.97), and UIA-related inpatient readmissions (OR 0.62, 95%CI 0.42-0.91) versus Group 2. Though bivariate analyses demonstrated lower total cost of care in Group 1 ($104,667 vs. $126,796, p=0.0018) versus Group 2, significance was lost in the GEE model (adjusted ratio of the mean 0.83, 95%CI 0.62-1.10). This evidenced that Group 1 endovascular treatment for UIA using the stent 10 was associated with significantly lower 12-month readmission versus the stent used with Group 2.

Inclusion criteria for the study included subjects ≥ 18 years old at the time of index hospital admission (i.e., first observed inpatient hospital admission). Exclusion criteria for each study included patients who had their index endovascular procedure at hospitals who did not continuously provide data to PHD in the 12-month pre-index period.

### Results of the Study

The unmatched cohort included 1,659 patients (616 Group 1 and 1,043 Group 2 [782 Neuroform stent and 261 LVIS stent]). In this cohort, Group 1 and Group 2 differed significantly in terms of marital status, payor status, bed size, hospital geographic location, hospital type, and comorbidities. Most notable was a higher proportion of CCI scores 2-3 in Group 1 versus Group 2 groups (71.6% vs. 63.7%, p=0.001) and higher prevalence of specific comorbidities including stroke/TIA (13.2% vs. 10.0%, p=0.047), peripheral vascular disease (71.4% vs. 57.1%, p<0.0001), and depression (17.4% vs. 13.0%, p=0.016) in Group 1 versus Group 2.

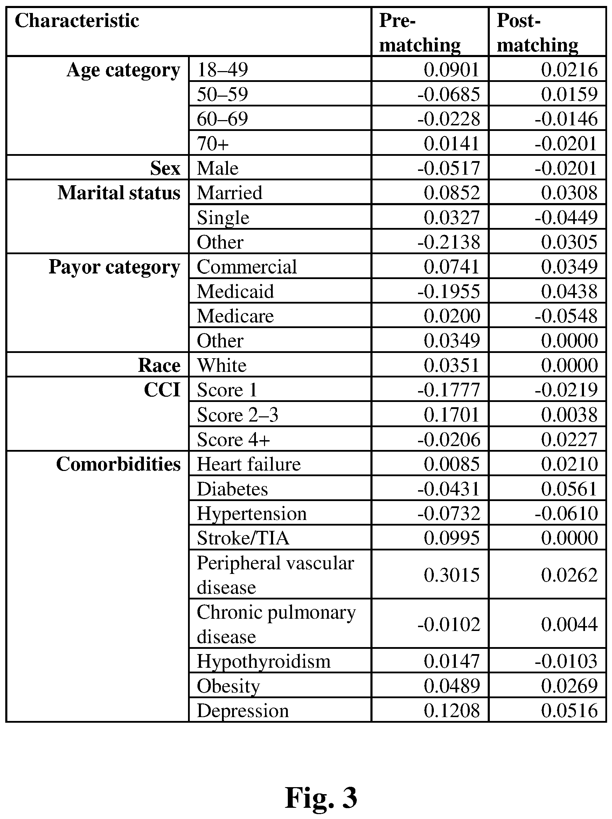

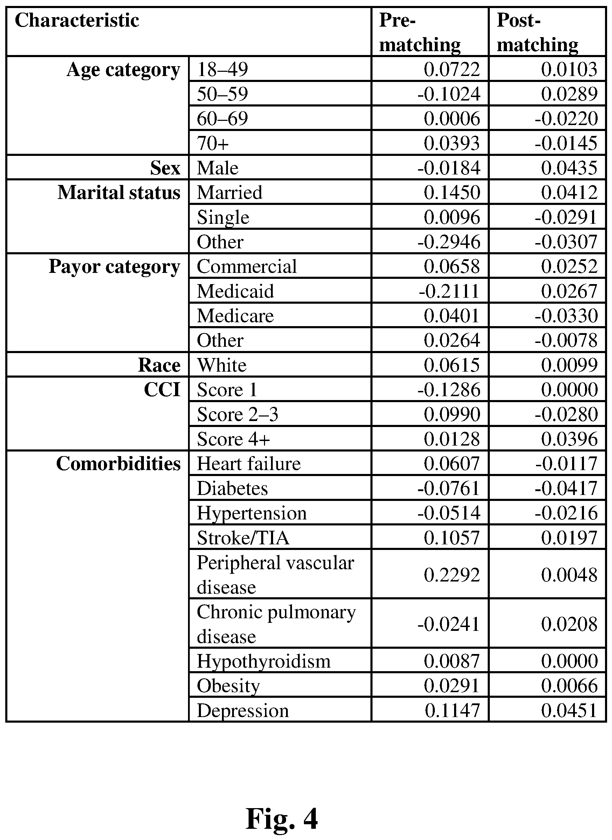

The results of propensity matching are detailed in Fig. 3. A total of 585 patient pairs were propensity matched for outcome comparison. Examination of SMDs revealed a perfect balance of covariates among the matched cohort, with no significant differences observed for any of the covariates. When considering only patients from hospitals that provided continuous data during the 12-month period after index hospital admission, 486 patients were matched to each group depicted in Fig. 4. Examination of SMDs for the matched cohort depicted a good balance, with a significant difference observed only for the "year" variable post-matching. Accordingly, GEE models comparing 12-month readmission and costs of care were adjusted for calendar "year" in addition to hospital-level clustering.

Bivariate and adjusted analyses of discharge status defined in terms of mortality and LOS were conducted on the propensity-matched cohort (n=585 per group) to compare Group 1 and Group 2. Discharge status did not significantly differ between Groups 1 and 2 in either analysis (Group 1 vs. Group 2, 1.0% vs. 0.5%, p=0.51; adjusted ratio of the mean 1.98, 95% CI 0.48-8.07). In contrast, LOS was significantly shorter in Group 1 compared to the comparator group in the bivariate analysis (1.89±2.07 vs. 2.11±2.81, p<0.0001), but did not emerge to be a statistically significantly difference in GEE analysis (adjusted ratio of the mean 0.90, 95% CI 0.73-1.10).

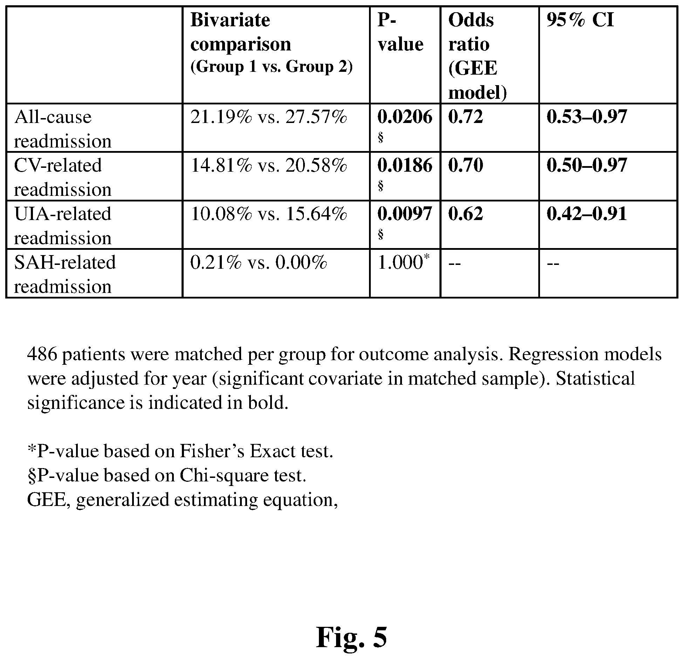

Analyses of 12-month follow-up outcomes were conducted on a cohort of propensity-matched patients from hospitals that continuously provided data to the PHD for 12 months after the index hospital admission (n=486 per group). With regard to inpatient readmission, Group 1 group exhibited lower rates of all-cause (21.2% vs. 27.6%, p=0.021), CV-related (14.8% vs. 20.6%, p=0.019), and UIA-related readmission (10.1% vs. 15.6%, p=0.010) compared to the comparator group in the bivariate analysis. Statistical significance was preserved after adjustment in the GEE analysis: Group 1 patients had 28% lower odds of 12-month all-cause inpatient readmission (OR 0.72, 95% CI 0.53-0.97), 30% lower odds of 12-month CV-related readmission (OR 0.70, 95% CI 0.50-0.97), and 38% lower odds of 12-month UIA-related readmission (OR 0.62, 95% CI 0.42-0.91) compared to Group 2, as shown in the table of Fig. 5.

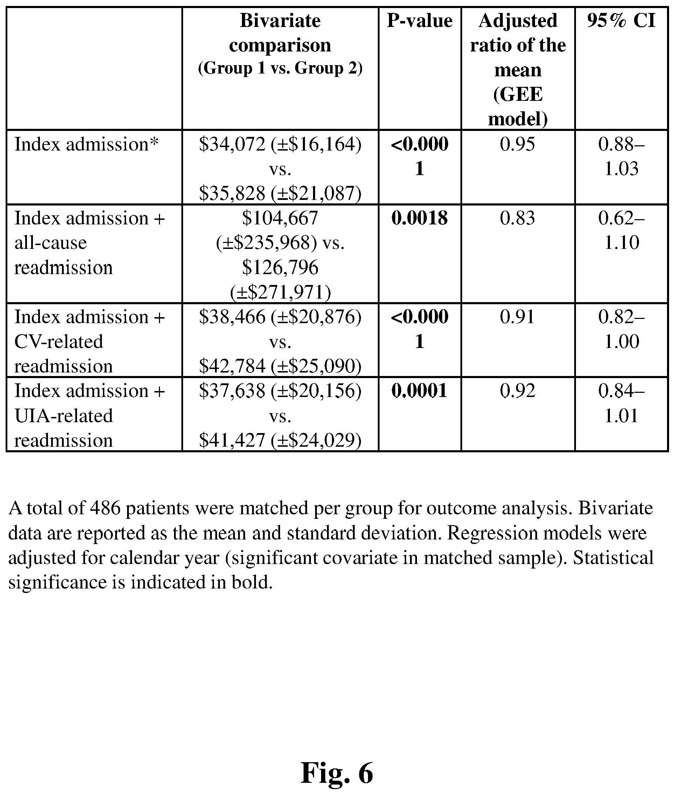

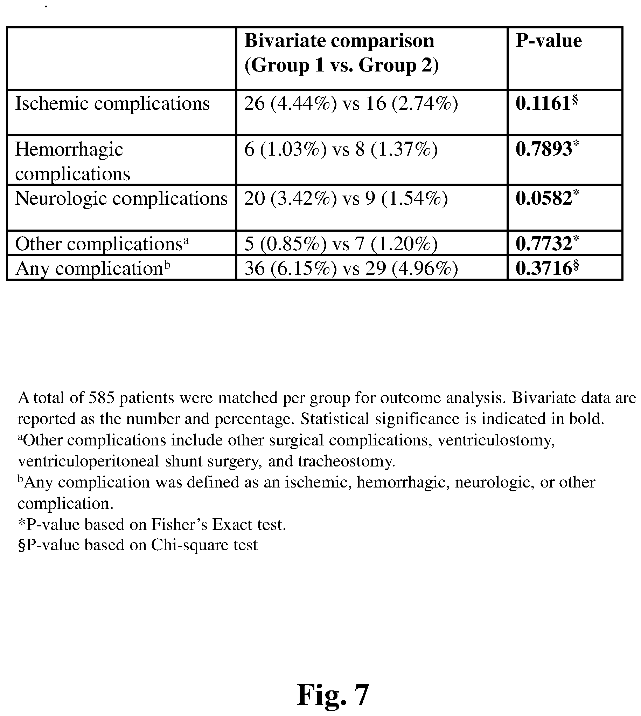

It was determined that mean cost of index admission was significantly lower in Group 1 than Group 2 in the bivariate analysis ($34,072 vs. $35,828, p<0.001). A similar trend was observed for total costs (index admission plus 12-month all-cause inpatient readmission), which were lower in Group 1 when considering all-cause readmission ($104,667 vs. $126,795, p=0.002), CV-related readmission ($38,466 vs. $42,784, p<0.0001), and UIA-related readmission ($37,638 vs. $41,427, p=0.001), as shown in Fig. 6. Bivariate analysis of complication rates between Group 1 and Group 2 groups (n=585) revealed no significant differences in the rates of total, ischemic, hemorrhagic, neurologic, or other complications, as shown in Fig. 7.

In sum, Group 1 was associated with a significantly lower rate of retreatment at least since patients treated in Group 1 had 28 to 38% lower odds of all-cause, CV-related, and UIA-related readmission compared to the comparator group. In the bivariate analysis, this difference was associated with a corresponding lower cost. The approximately 38% lower likelihood of UIA-related readmission in the subsequent 12-month period post-endovascular procedure for Group 1 versus Group 2 signifies a clinical benefit accrued with the use of stent 10 with Group 1. Regarding costs, endovascular procedure costs were $1,756 lower in Group 1 versus Group 2. When examining the total cost of care, calculated as the sum of index endovascular procedure costs and 12-month all-cause inpatient admission costs, Group 1 was associated with a cost savings of $22,129 compared to Group 2. When limiting the total cost of care to include only the index procedure and 12-month UIA-related readmission costs, this cost difference was $3,789 in favor of Group 1. Notably, these differences approached significance in the GEE model. These results indicate a potential economic benefit associated with use the stent 10 of Group 1 versus those of Group 2.

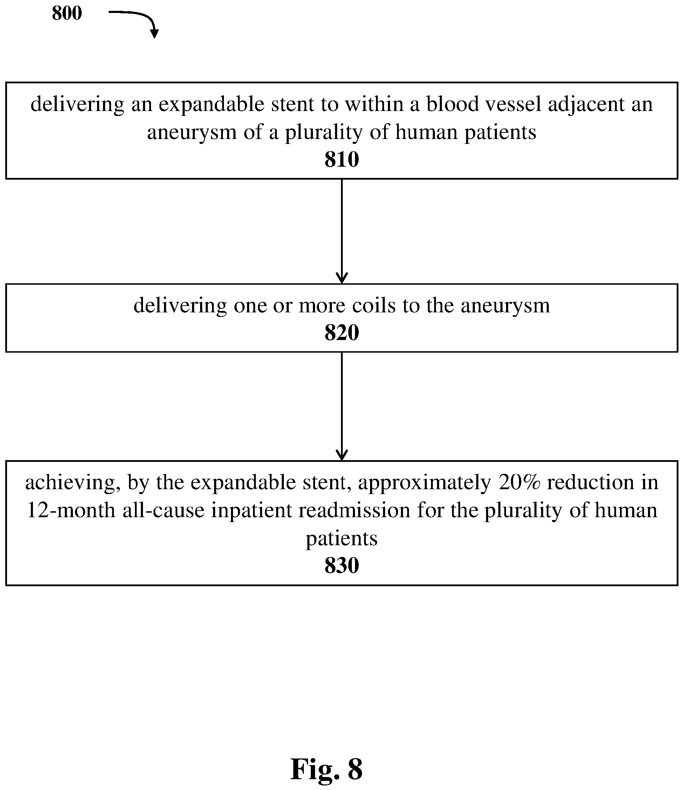

FIG. 8 depicts a method or use 800 and can include 810 delivering an expandable stent to within a blood vessel adjacent an aneurysm of a plurality of human patients; 820 delivering one or more coils to the aneurysm; and 830 achieving, by the expandable stent, approximately 20% reduction in 12-month all-cause inpatient readmission for the plurality of human patients. Method or use 800 can end after step 830.

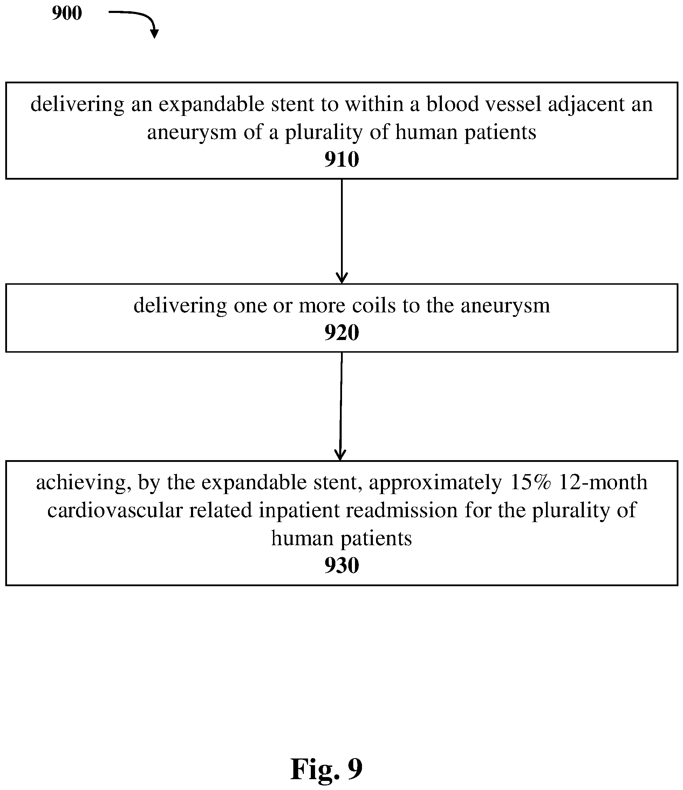

FIG. 9 depicts a method or use 900 and can include 910 delivering an expandable stent to within a blood vessel adjacent an aneurysm of a plurality of human patients; 920 delivering one or more coils to the aneurysm; and 930 achieving, by the expandable stent, approximately 15% reduction in 12-month cardiovascular related inpatient readmission for the plurality of human patients. Method or use 900 can end after step 930. In other embodiments, additional steps according to the examples described above can be performed.

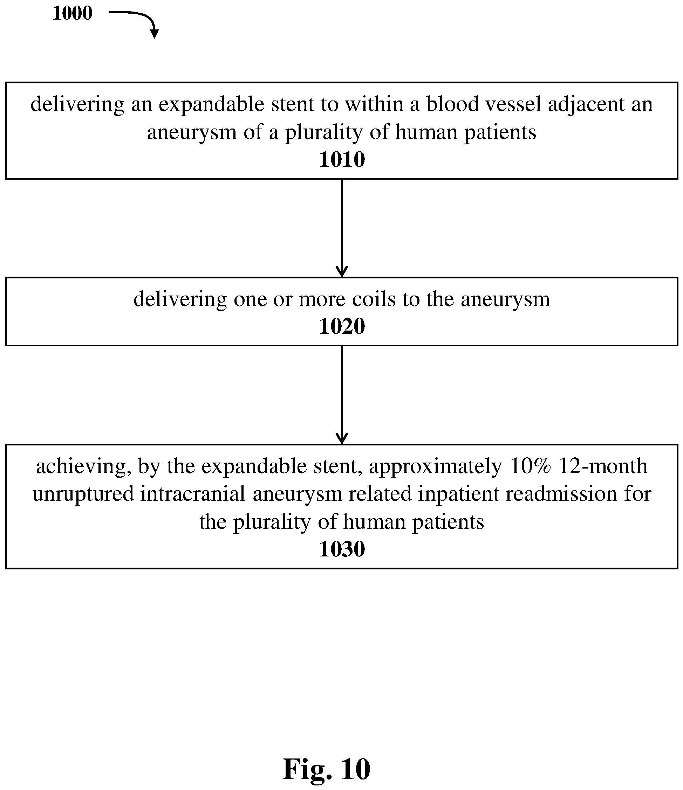

FIG. 10 depicts a method or use 1000 and can include 1010 delivering an expandable stent to within a blood vessel adjacent an aneurysm of a plurality of human patients; 1020 delivering one or more coils to the aneurysm; and 1030 achieving, by the expandable stent, approximately 10% reduction in 12-month unruptured intracranial aneurysm related inpatient readmission for the plurality of human patients. Method or use 1000 can end after step 1030. In other embodiments, additional steps according to the examples described above can be performed.

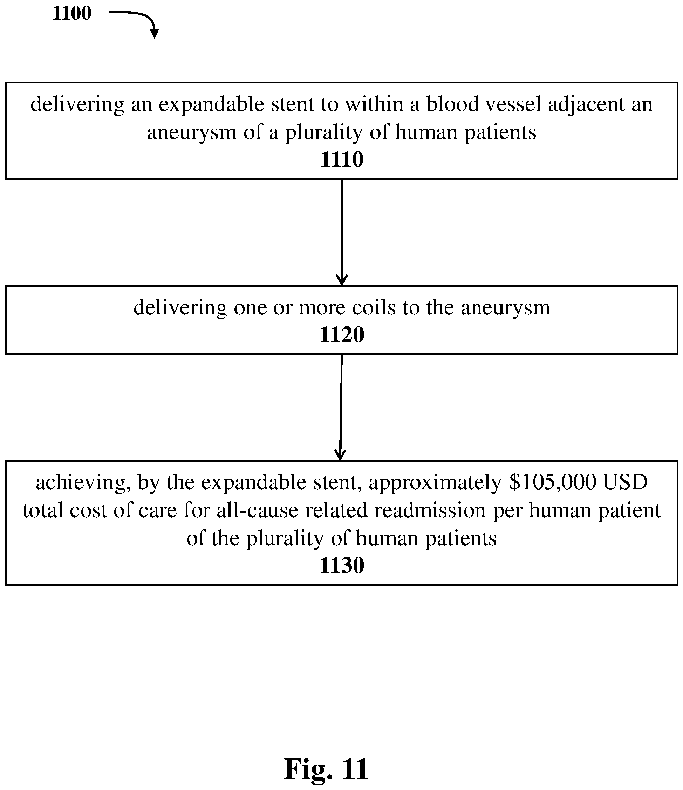

FIG. 11 depicts a method or use 1100 and can include 1110 delivering an expandable stent to within a blood vessel adjacent an aneurysm of a plurality of human patients; 1120 delivering one or more coils to the aneurysm; and 1130 achieving, by the expandable stent, approximately $105,000 USD total cost of care for all-cause related readmission per human patient of the plurality of human patients. Method or use 1100 can end after step 1130. In other embodiments, additional steps according to the examples described above can be performed.

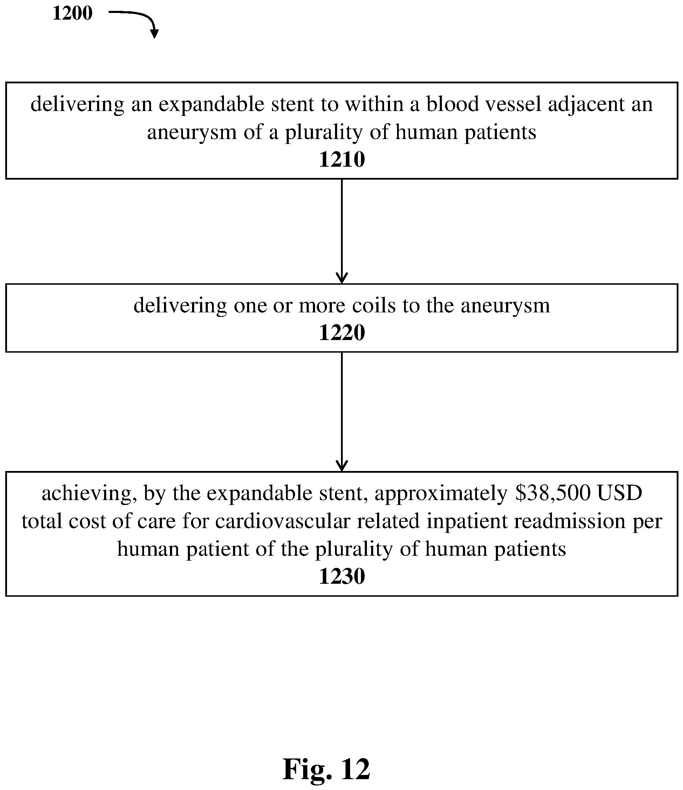

FIG. 12 depicts a method or use 1200 and can include 1210 delivering an expandable stent to within a blood vessel adjacent an aneurysm of a plurality of human patients; 1220 delivering one or more coils to the aneurysm; and 1230 achieving, by the expandable stent, approximately $38,500 USD total cost of care for cardiovascular related inpatient readmission per human patient of the plurality of human patients. Method or use 1200 can end after step 1230. In other embodiments, additional steps according to the examples described above can be performed.

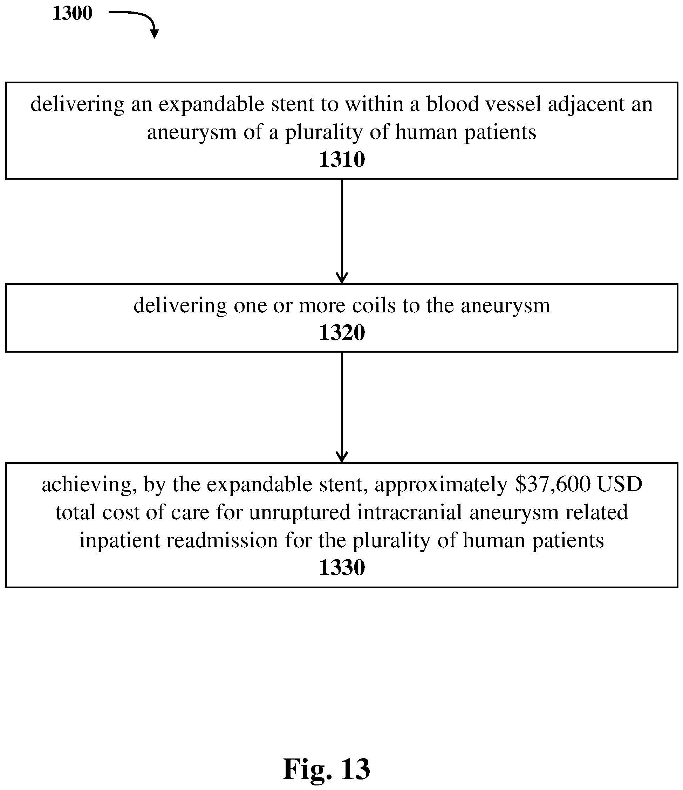

FIG. 13 depicts a method or use 1300 and can include 1310 delivering an expandable stent to within a blood vessel adjacent an aneurysm of a plurality of human patients; 1320 delivering one or more coils to the aneurysm; and 1330 achieving, by the expandable stent, approximately $37,600 USD total cost of care for unruptured intracranial aneurysm related inpatient readmission for the plurality of human patients. Method or use 1300 can end after step 1330. In other embodiments, additional steps according to the examples described above can be performed.

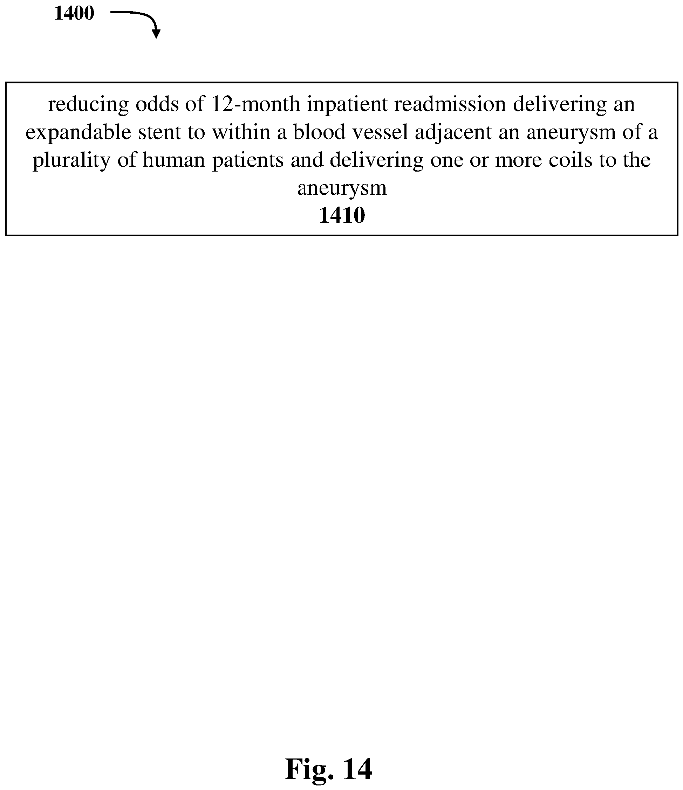

FIG. 14 depicts a method or use 1400 and can include 1410 reducing odds of 12-month inpatient readmission by delivering an expandable stent to within a blood vessel adjacent an aneurysm of a plurality of human patients and delivering one or more coils to the aneurysm. Method or use 1400 can end after step 1410. In other embodiments, additional steps according to the examples described above can be performed.

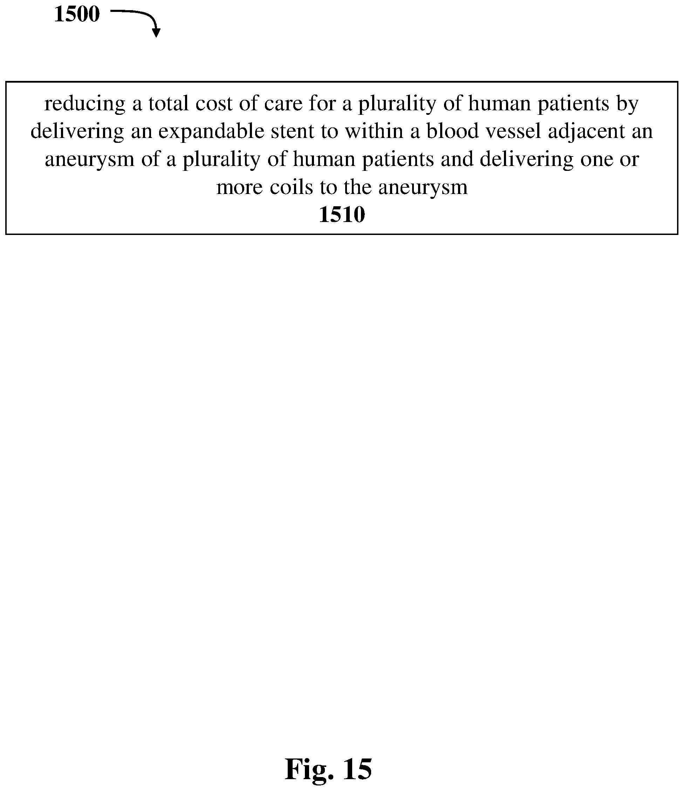

FIG. 15 depicts a method or use 1500 and can include 1510 reducing a total cost of care for a plurality of human patients by delivering an expandable stent to within a blood vessel adjacent an aneurysm of a plurality of human patients and delivering one or more coils to the aneurysm. Method or use 1500 can end after step 1510. In other embodiments, additional steps according to the examples described above can be performed.

The self-expanding braided stent 10 and related methods of use of this disclosure, demonstrated high rates of substantial complete aneurysm occlusion and functional independence in patients with hemorrhagic events (e.g., an aneurysm). The specific configurations, choice of materials and the size and shape of various elements can be varied according to particular design specifications or constraints requiring a system or method constructed according to the principles of the disclosed technology. Such changes are intended to be embraced within the scope of the disclosed technology. The presently disclosed embodiments, therefore, are considered in all respects to be illustrative and not restrictive. It will therefore be apparent from the foregoing that while particular forms of the disclosure have been illustrated and described, various modifications can be made without departing from the spirit and scope of the disclosure and all changes that come within the meaning and range of equivalents thereof are intended to be embraced therein. Any or all of the methods or uses described herein may be not methods for treatment of the human or animal body by surgery or therapy. For example, the methods described can take place after any surgical or therapeutic method has taken place i.e. the methods described do not encompass within their scope any method of treatment by surgery or therapy because there is a temporal gap between the methods described and any methods of treatment of the human or animal body by surgery or therapy. The methods or uses described are also not methods for treatment of the human or animal body by diagnosis.

The following clauses list non-limiting embodiments of the disclosure.
1. A method or use comprising:
   reducing odds of 12-month inpatient readmission by delivering an expandable stent to within a blood vessel adjacent an aneurysm of each of a first plurality of human patients in a first group and delivering one or more coils to the aneurysm of each of the first plurality of human patients in the first group.
2. The method or use according to Clause 1,
   wherein a second group is treated by a second clinically approved stent, and
   wherein odds of 12-month all-cause readmission are at least approximately 28% lower for the first group versus the second group, the odds being determined by a Generalized estimating equations (GEE) model.
3. The method or use according to Clause 1,
   wherein a second group is treated by a second clinically approved stent, and
   wherein odds of 12-month cardiovascular related readmission are at least approximately 30% lower for the first group versus the second group, the odds being determined by a Generalized estimating equations (GEE) model.
4. The method or use according to Clause 1,
   wherein a second group is treated by a second clinically approved stent, and
   wherein odds of 12-month unruptured intracranial aneurysm related readmission are at least approximately 38% lower for the first group versus the second group, the odds being determined by a Generalized estimating equations (GEE) model.
5. The method or use according to Clause 1, further comprising:
   achieving, by the expandable stent, approximately 20% 12-month all-cause inpatient readmission for the first plurality of human patients,
6. The method or use according to Clause 1, further comprising:
   achieving, by the expandable stent, approximately 15% 12-month cardiovascular related inpatient readmission for the first plurality of human patients,
7. The method or use according to Clause 1, further comprising:
   achieving, by the expandable stent, approximately 10% 12-month unruptured intracranial aneurysm related inpatient readmission for the first plurality of human patients.
8. The method or use according to Clause 1, the first plurality of human patients comprising at least approximately 486 human patients from a plurality of different hospital sites.
9. The method or use according to Clause 1, the first plurality of human patients comprising inclusion criteria defined as patients who underwent an inpatient endovascular procedure for unruptured intracranial aneurysm between 2011-2018 were identified from the Premier Healthcare Database.
10. The method or use according to Clause 1, the first plurality of human patients comprising inclusion criteria defined as patients ≥ 18 years old at the time of index hospital admission.
11. The method or use according to Clause 1, the first plurality of human patients comprising exclusion criteria defined as patients who had their index endovascular procedure at hospitals who did not continuously provide data to PHD in the 12-month pre-index period.
12. The method or use according to Clause 1, the expandable stent comprising at least one anchor member interlocked within at least one gap along a core member of a corresponding delivery system, the at least one gap formed by spaces between a plurality of cylindrical members disposed on the core member.
13. The method or use according to Clause 12, further comprising:
   mounting the expandable stent on at least one cylindrical member along the core member of the delivery system, the delivery system comprising a deployment catheter disposed about the stent maintaining the stent in a constrained configuration.
14. The method or use according to Clause 13, further comprising:
   moving the deployment catheter proximally allowing said stent to begin expanding within the vessel.
15. The method or use according to Clause 14, further comprising:
   further moving the deployment catheter proximally allowing the stent to further deploy causing the vessel to increase luminal dilation.
16. The method or use according to Clause 14, further comprising:
   removing said delivery system from said blood vessel.
17. A method or use, comprising:
   delivering an expandable stent to within a blood vessel adjacent an aneurysm of a plurality of human patients;
   delivering one or more coils to the aneurysm; and
   achieving, by the expandable stent, approximately 20% 12-month all-cause inpatient readmission for the plurality of human patients.
18. A method or use, comprising:
   delivering an expandable stent to within a blood vessel adjacent an aneurysm of a plurality of human patients;
   delivering one or more coils to the aneurysm; and
   achieving, by the expandable stent, approximately 15% 12-month cardiovascular related inpatient readmission for the plurality of human patients.
19. A method or use, comprising:
   reducing a total cost of care for a first plurality of human patients in a first group by delivering an expandable stent to within a blood vessel adjacent an aneurysm of each of the first plurality of human patients and delivering one or more coils to the aneurysm of each of the first plurality of human patients.
20. The method or use according to Clause 19,
   wherein a second group is treated by a second clinically approved stent,
   wherein a total cost of care for all-cause related readmission per human patient of the plurality of human patients is approximately $105,000 USD for the first group and approximately $127,000 USD for the second group, and
   wherein the total cost of care is adjusted for medical inflation and reported in 2018 US dollars ($).
21. The method or use according to Clause 19,
   wherein a second group is treated by a second clinically approved stent,
   wherein a total cost of care for cardiovascular related inpatient readmission per human patient of the plurality of human patients is approximately $38,500 USD for the first group and approximately $42,800 USD for the second group, and
   wherein the total cost of care is adjusted for medical inflation and reported in 2018 US dollars ($).
22. The method or use according to Clause 19,
   wherein a second group is treated by a second clinically approved stent,
   wherein a total cost of care for unruptured intracranial aneurysm related inpatient readmission per human patient of the plurality of human patients is approximately $37,600 USD for the first group and approximately $41,400 USD for the second group, and
   wherein the total cost of care is adjusted for medical inflation and reported in 2018 US dollars ($).
23. The method or use according to Clause 19, further comprising:
   achieving, by the expandable stent, a total cost of care selected from a plurality of total costs of care, the total cost of care being adjusted for medical inflation and reported in 2018 US dollars ($), the plurality of total costs of care consisting of:
      approximately $105,000 USD total cost of care for all-cause related readmission per human patient of the first plurality of human patients,
      approximately $38,500 USD total cost of care for cardiovascular related inpatient readmission per human patient of the first plurality of human patients, and
      approximately $37,600 USD total cost of care for unruptured intracranial aneurysm related inpatient readmission for the first plurality of human patients.
24. The method or use according to Clause 19, the first plurality of human patients comprising at least approximately 486 human patients from a plurality of different hospital sites.
25. The method or use according to Clause 19, the first plurality of human patients comprising inclusion criteria defined as patients who underwent an inpatient endovascular procedure for unruptured intracranial aneurysm between 2011-2018 were identified from the Premier Healthcare Database.
26. The method or use according to Clause 19, the first plurality of human patients comprising inclusion criteria defined as patients ≥ 18 years old at the time of index hospital admission.
27. The method or use according to Clause 19, the first plurality of human patients comprising exclusion criteria defined as patients who had their index endovascular procedure at hospitals who did not continuously provide data to PHD in the 12-month pre-index period.
28. The method or use according to Clause 19, the expandable stent comprising at least one anchor member interlocked within at least one gap along a core member of a corresponding delivery system, the at least one gap formed by spaces between a plurality of cylindrical members disposed on the core member.
29. The method or use according to Clause 28, further comprising:
   mounting the expandable stent on at least one cylindrical member along the core member of the delivery system, the delivery system comprising a deployment catheter disposed about the stent maintaining the stent in a constrained configuration.
30. The method or use according to Clause 29, further comprising:
   moving the deployment catheter proximally allowing said stent to begin expanding within the vessel.
31. The method or use according to Clause 30, further comprising:
   further moving the deployment catheter proximally allowing the stent to further deploy causing the vessel to increase luminal dilation.
32. The method or use according to Clause 30, further comprising:
   removing said delivery system from said blood vessel.

### Embodiments

1. A method or use comprising:
   reducing odds of 12-month inpatient readmission by delivering an expandable stent to within a blood vessel adjacent an aneurysm of each of a first plurality of human patients in a first group and delivering one or more coils to the aneurysm of each of the first plurality of human patients in the first group.
2. The method or use according to Embodiment 1,
   wherein a second group is treated by a second clinically approved stent, and
   wherein odds of 12-month all-cause readmission are at least approximately 28% lower for the first group versus the second group, the odds being determined by a Generalized estimating equations (GEE) model.
3. The method or use according to Embodiment 1,
   wherein a second group is treated by a second clinically approved stent, and
   wherein odds of 12-month cardiovascular related readmission are at least approximately 30% lower for the first group versus the second group, the odds being determined by a Generalized estimating equations (GEE) model.
4. The method or use according to Embodiment 1,
   wherein a second group is treated by a second clinically approved stent, and
   wherein odds of 12-month unruptured intracranial aneurysm related readmission are at least approximately 38% lower for the first group versus the second group, the odds being determined by a Generalized estimating equations (GEE) model.
5. The method or use according to Embodiment 1, further comprising:
   achieving, by the expandable stent, approximately 20% 12-month all-cause inpatient readmission for the first plurality of human patients,
6. The method or use according to Embodiment 1, further comprising:
   achieving, by the expandable stent, approximately 15% 12-month cardiovascular related inpatient readmission for the first plurality of human patients,
7. The method or use according to Embodiment 1, further comprising:
   achieving, by the expandable stent, approximately 10% 12-month unruptured intracranial aneurysm related inpatient readmission for the first plurality of human patients.
8. The method or use according to Embodiment 1, the first plurality of human patients comprising at least approximately 486 human patients from a plurality of different hospital sites.
9. The method or use according to Embodiment 1, the first plurality of human patients comprising inclusion criteria defined as patients who underwent an inpatient endovascular procedure for unruptured intracranial aneurysm between 2011-2018 were identified from the Premier Healthcare Database.
10. The method or use according to Embodiment 1, the first plurality of human patients comprising inclusion criteria defined as patients ≥ 18 years old at the time of index hospital admission.
11. The method or use according to Embodiment 1, the first plurality of human patients comprising exclusion criteria defined as patients who had their index endovascular procedure at hospitals who did not continuously provide data to PHD in the 12-month pre-index period.
12. The method or use according to Embodiment 1, the expandable stent comprising at least one anchor member interlocked within at least one gap along a core member of a corresponding delivery system, the at least one gap formed by spaces between a plurality of cylindrical members disposed on the core member.
13. The method or use according to Embodiment 12, further comprising:
   mounting the expandable stent on at least one cylindrical member along the core member of the delivery system, the delivery system comprising a deployment catheter disposed about the stent maintaining the stent in a constrained configuration.
14. The method or use according to Embodiment 13, further comprising: moving
   the deployment catheter proximally allowing said stent to begin expanding within the vessel.
15. The method or use according to Embodiment 14, further comprising:
   further moving the deployment catheter proximally allowing the stent to further deploy causing the vessel to increase luminal dilation.
16. The method or use according to Embodiment 14, further comprising:
   removing said delivery system from said blood vessel.
17. A method or use, comprising:
   delivering an expandable stent to within a blood vessel adjacent an aneurysm of plurality of human patients;
   delivering one or more coils to the aneurysm; and
   achieving, by the expandable stent, approximately 20% 12-month all-cause inpatient readmission for the plurality of human patients.
18. A method or use, comprising:
   delivering an expandable stent to within a blood vessel adjacent an aneurysm of a plurality of human patients;
   delivering one or more coils to the aneurysm; and
   achieving, by the expandable stent, approximately 15% 12-month cardiovascular related inpatient readmission for the plurality of human patients.

### Aspects

1. An apparatus comprising:
   an expandable stent configured for delivery to within a blood vessel adjacent an aneurysm of each of a first plurality of human patients in a first group; and
   one or more coils configured for delivery to the aneurysm of each of the first plurality of human patients in the first group,
   wherein delivery of the expandable stent to the first plurality of human patients in the first group reduces odds of 12-month inpatient readmission in the first plurality of human patients in the first group.
2. The apparatus according to aspect 1, wherein delivery of the expandable stent to the first plurality of human patients in the first group means that odds of 12-month all-cause readmission are at least approximately 28% lower for the first group versus a second group treated by a second clinically approved stent, the odds being determined by a Generalized estimating equations (GEE) model, and/or
   wherein delivery of the expandable stent to the first plurality of human patients in the first group means that odds of 12-month cardiovascular readmission are at least approximately 30% lower for the first group versus a second group treated by a second clinically approved stent, the odds being determined by a Generalized estimating equations (GEE) model, and/or
   wherein delivery of the expandable stent to the first plurality of human patients in the first group means that odds of 12-month unruptured intracranial aneurysm related readmission are at least approximately 38% lower for the first group versus a second group treated by a second clinically approved stent, the odds being determined by a Generalized estimating equations (GEE) model.
3. The apparatus according to aspect 1, wherein the expandable stent achieves approximately 20% 12-month all-cause inpatient readmission for the first plurality of human patients, and/or
   wherein the expandable stent achieves approximately 15% 12-month cardiovascular related inpatient readmission for the first plurality of human patients, and/or
   wherein the expandable stent achieves approximately 10% 12-month unruptured intracranial aneurysm related inpatient readmission for the first plurality of human patients.
4. A method or use comprising:
   reducing odds of 12-month inpatient readmission in a first plurality of human patients each of whom has had an expandable stent delivered to within a blood vessel adjacent an aneurysm of each of a first plurality of human patients in a first group and each of whom has had one or more coils delivered to the aneurysm of each of the first plurality of human patients in the first group.
5. The method or use according to aspect 4,
   wherein a second group has been treated by a second clinically approved stent, and
   wherein odds of 12-month all-cause readmission are at least approximately 28% lower for the first group versus the second group, the odds being determined by a Generalized estimating equations (GEE) model, and/or
   wherein a second group has been treated by a second clinically approved stent, and
   wherein odds of 12-month cardiovascular related readmission are at least approximately 30% lower for the first group versus the second group, the odds being determined by a Generalized estimating equations (GEE) model, and/or
   wherein a second group has been treated by a second clinically approved stent, and
   wherein odds of 12-month unruptured intracranial aneurysm related readmission are at least approximately 38% lower for the first group versus the second group, the odds being determined by a Generalized estimating equations (GEE) model.
6. The method or use according to aspect 4, further comprising:
   achieving, by the expandable stent, approximately 20% 12-month all-cause inpatient readmission for the first plurality of human patients, and/or
   achieving, by the expandable stent, approximately 15% 12-month cardiovascular related inpatient readmission for the first plurality of human patients, and/or
   achieving, by the expandable stent, approximately 10% 12-month unruptured intracranial aneurysm related inpatient readmission for the first plurality of human patients.
7. The apparatus according to aspect 1 or the method or use according to aspect 4, the first plurality of human patients comprising at least approximately 486 human patients from a plurality of different hospital sites, and/or
   the first plurality of human patients comprising inclusion criteria defined as patients who underwent an inpatient endovascular procedure for unruptured intracranial aneurysm between 2011-2018 were identified from the Premier Healthcare Database, and/or
   the first plurality of human patients comprising inclusion criteria defined as patients ≥ 18 years old at the time of index hospital admission, and/or
   the first plurality of human patients comprising exclusion criteria defined as patients who had their index endovascular procedure at hospitals who did not continuously provide data to PHD in the 12-month pre-index period.
8. The apparatus according to aspect 1 or the method or use according to aspect 4, the expandable stent comprising at least one anchor member interlocked within at least one gap along a core member of a corresponding delivery system, the at least one gap formed by spaces between a plurality of cylindrical members disposed on the core member.
9. The apparatus according to aspect 8, wherein the expandable stent is configured for mounting on at least one cylindrical member along the core member of the delivery system, the delivery system comprising a deployment catheter disposed about the stent maintaining the stent in a constrained configuration.
10. The apparatus according to aspect 9, is wherein the deployment catheter is configured to be moved proximally allowing said stent to begin expanding within the vessel.
11. The apparatus according to aspect 10, wherein the deployment catheter is configured to be moved proximally allowing the stent to further deploy causing the vessel to increase luminal dilation.
12. The apparatus according to aspect 10, wherein said delivery system is configured for removal from said blood vessel.
13. An apparatus, comprising:
   an expandable stent configured for delivery to within a blood vessel adjacent an aneurysm of a plurality of human patients; and
   one or more coils configured for delivery to the aneurysm,
   wherein delivery of the expandable stent to the plurality of human patients achieves approximately 20% 12-month all-cause inpatient readmission for the plurality of human patients.
14. An apparatus, comprising:
   an expandable stent configured for delivery to within a blood vessel adjacent an aneurysm of a plurality of human patients; and
   one or more coils configured for delivery to the aneurysm,
   wherein delivery of the expandable stent to the plurality of human patients achieves approximately 15% 12-month cardiovascular related inpatient readmission for the plurality of human patients.

## Claims

1. An apparatus comprising:
an expandable stent configured for delivery to within a blood vessel adjacent an aneurysm of each of a first plurality of human patients in a first group; and
one or more coils configured for delivery to the aneurysm of each of the first plurality of human patients in the first group,
wherein delivery of the expandable stent to the first plurality of human patients in the first group reduces odds of 12-month inpatient readmission in the first plurality of human patients in the first group.

2. The apparatus according to claim 1, wherein delivery of the expandable stent to the first plurality of human patients in the first group means that odds of 12-month all-cause readmission are at least approximately 28% lower for the first group versus a second group treated by a second clinically approved stent, the odds being determined by a Generalized estimating equations (GEE) model, and/or
wherein delivery of the expandable stent to the first plurality of human patients in the first group means that odds of 12-month cardiovascular readmission are at least approximately 30% lower for the first group versus a second group treated by a second clinically approved stent, the odds being determined by a Generalized estimating equations (GEE) model, and/or wherein delivery of the expandable stent to the first plurality of human patients in the first group means that odds of 12-month unruptured intracranial aneurysm related readmission are at least approximately 38% lower for the first group versus a second group treated by a second clinically approved stent, the odds being determined by a Generalized estimating equations (GEE) model.

3. The apparatus according to claim 1, wherein the expandable stent achieves approximately 20% 12-month all-cause inpatient readmission for the first plurality of human patients, and/or
wherein the expandable stent achieves approximately 15% 12-month cardiovascular related inpatient readmission for the first plurality of human patients, and/or
wherein the expandable stent achieves approximately 10% 12-month unruptured intracranial aneurysm related inpatient readmission for the first plurality of human patients.

4. A method or use comprising:
reducing odds of 12-month inpatient readmission in a first plurality of human patients each of whom has had an expandable stent delivered to within a blood vessel adjacent an aneurysm of each of a first plurality of human patients in a first group and each of whom has had one or more coils delivered to the aneurysm of each of the first plurality of human patients in the first group.

5. The method or use according to claim 4,
wherein a second group has been treated by a second clinically approved stent, and
wherein odds of 12-month all-cause readmission are at least approximately 28% lower for the first group versus the second group, the odds being determined by a Generalized estimating equations (GEE) model, and/or
wherein a second group has been treated by a second clinically approved stent, and
wherein odds of 12-month cardiovascular related readmission are at least approximately 30% lower for the first group versus the second group, the odds being determined by a Generalized estimating equations (GEE) model, and/or
wherein a second group has been treated by a second clinically approved stent, and
wherein odds of 12-month unruptured intracranial aneurysm related readmission are at least approximately 38% lower for the first group versus the second group, the odds being determined by a Generalized estimating equations (GEE) model.

6. The method or use according to claim 4, further comprising:
achieving, by the expandable stent, approximately 20% 12-month all-cause inpatient readmission for the first plurality of human patients, and/or
achieving, by the expandable stent, approximately 15% 12-month cardiovascular related inpatient readmission for the first plurality of human patients, and/or
achieving, by the expandable stent, approximately 10% 12-month unruptured intracranial aneurysm related inpatient readmission for the first plurality of human patients.

7. The apparatus according to claim 1 or the method or use according to claim 4, the first plurality of human patients comprising at least approximately 486 human patients from a plurality of different hospital sites, and/or
the first plurality of human patients comprising inclusion criteria defined as patients who underwent an inpatient endovascular procedure for unruptured intracranial aneurysm between 2011-2018 were identified from the Premier Healthcare Database, and/or
the first plurality of human patients comprising inclusion criteria defined as patients ≥ 18 years old at the time of index hospital admission, and/or
the first plurality of human patients comprising exclusion criteria defined as patients who had their index endovascular procedure at hospitals who did not continuously provide data to PHD in the 12-month pre-index period.

8. The apparatus according to claim 1 or the method or use according to claim 4, the expandable stent comprising at least one anchor member interlocked within at least one gap along a core member of a corresponding delivery system, the at least one gap formed by spaces between a plurality of cylindrical members disposed on the core member.

9. The apparatus according to claim 8, wherein the expandable stent is configured for mounting on at least one cylindrical member along the core member of the delivery system, the delivery system comprising a deployment catheter disposed about the stent maintaining the stent in a constrained configuration.

10. The apparatus according to claim 9, wherein the deployment catheter is configured to be moved proximally allowing said stent to begin expanding within the vessel.

11. The apparatus according to claim 10, wherein the deployment catheter is configured to be moved proximally allowing the stent to further deploy causing the vessel to increase luminal dilation.

12. The apparatus according to claim 10, wherein said delivery system is configured for removal from said blood vessel.

13. An apparatus, comprising:
an expandable stent configured for delivery to within a blood vessel adjacent an aneurysm of a plurality of human patients; and
one or more coils configured for delivery to the aneurysm,
wherein delivery of the expandable stent to the plurality of human patients achieves approximately 20% 12-month all-cause inpatient readmission for the plurality of human patients.

14. An apparatus, comprising:
an expandable stent configured for delivery to within a blood vessel adjacent an aneurysm of a plurality of human patients; and
one or more coils configured for delivery to the aneurysm,
wherein delivery of the expandable stent to the plurality of human patients achieves approximately 15% 12-month cardiovascular related inpatient readmission for the plurality of human patients.
